Europäisches Patentamt

European Patent Office

Office européen des brevets

(19)

(11) Numéro de publication: **0 077 713 B1**

(12) **FASCICULE DE BREVET EUROPEEN**

(45) Date de publication du fascicule du brevet:
**19.02.86**

(51) Int. Cl.⁴: **A 61 F 13/06,** A 43 B 7/00

(21) Numéro de dépôt: **82401858.4**

(22) Date de dépôt: **08.10.82**

(54) **Chaussure, notamment pour patients ayant subi une intervention chirurgicale au niveau de l'avant-pied.**

(30) Priorité: **09.10.81 FR 8119104**

(43) Date de publication de la demande:
**27.04.83 Bulletin 83/17**

(45) Mention de la délivrance du brevet:
**19.02.86 Bulletin 86/8**

(84) Etats contractants désignés:
**AT BE CH DE GB IT LI LU NL SE**

(56) Documents cités:
**FR - A - 2 097 294**
**FR - A - 2 127 196**
**US - A - 2 509 821**
**US - A - 3 584 402**
**US - A - 3 905 135**

(73) Titulaire: **Barouk, Louis Samuel, Chemin de la Roche,
F-33370 Yvrac (FR)**
Titulaire: **ETABLISSEMENTS MAYZAUD Maurice, Rue
Aimé-Cotton, Z.A.C. Brive Est FR-19100 Brive (FR)**

(72) Inventeur: **Barouk, Louis Samuel, Chemin de la Roche,
F-33370 Yvrac (FR)**
Inventeur: **Mayzaud, Maurice, Rue Aimé-Cotton Zac
Drive Est, F-19100 Brive (FR)**

(74) Mandataire: **Chameroy, Claude et al, c/o Cabinet
Malemont 42, avenue du Président Wilson, F-75116 Paris
(FR)**

## Description

La présente invention concerne une chaussure, notamment une chaussure postopératoire pour patients ayant subi une intervention chirurgicale au niveau de l'avant-pied, cette chaussure étant ouverte à l'avant et comprenant une tige comportant deux éléments lateraux et une semelle d'épaisseur inégale.

On sait que les opérations chirurgicales de l'avant-pied, qui représentent plus de 75 % des interventions des chirurgiens podologues, obligent pendant le premier mois de convalescence, par contre, l'arrière-pied qui n'est pas douloureux, peut parfaitement servir de point d'appui. Toutes les personnes atteintes d'une quelconque affection de l'avant-pied, non forcément consécutive à une opération chirurgicale, sont d'ailleurs confrontées à ce problème.

Pour le résoudre, plusieurs possibilités sont offertes. L'une d'entre elles consiste à ne pas du tout se servir du pied opéré ou atteint d'une affection et à marcher à l'aide de cannes anglaises ou de béquilles en prenant appui sur l'autre pied. Il est inutile de dire que cette façon de marcher est fort contraignante et inconfortable.

Une seconde possibilité revient tout simplement à marcher sur le talon du pied opéré ou atteint d'une affection, éventuellement par l'intermédiaire d'un plâtre moulé. Dans ce cas aussi, la position de marche est fort inconfortable et peut entraîner un déséquilibre surtout chez les personnes agées. La difficulté de déplacement se trouve en outre aggravée lorsque l'intervention chirurgicale ou l'affection touche les deux pieds.

On connaît par ailleurs, d'après la demande de brevet français FR-A-2 127 196 et le brevet américain US-A-3 905 135, des chaussures du type spécifié en préambule, qui bien qu'ayant essentiellement une fonction orthopédique, peuvent être utilisées comme chaussures post-opératoires permettant au patient de marcher sans avoir à se servir de cannes anglaises, de béquilles ou d'un plâtre moulé.

Toutefois, ces chaussures connues présentent l'inconvénient d'envelopper la plus grande partie de l'avant-pied qui, de ce fait, se trouve soumis au cours de la marche à des sollicitations ou à des efforts provoquant bien souvent des douleurs insupportables pour le patient.

La présente invention se propose de remédier à ces inconvénients et pour ce faire, elle a pour objet une chaussure du type spécifié en préambule, qui se caractérise en ce que la tige avec ses deux éléments latéraux qui se rejoignent à leurs parties inférieures de manière à former une première sont dimensionnés pour n'envelopper que l'arrière-pied du patient et reposent sur ladite semelle qui est nettement plus épaisse à l'avant qu'à l'arrière de la tige.

Grâce à la structure particulière de cette chaussure, l'avant-pied s'etend totalement à l'extérieur de la chaussure et de ce fait n'est soumis à aucune sollicitation ou effort de la part de celle-ci. En outre, de par la forme de sa semelle plus épaisse à l'avant, la chaussure selon l'invention maintient le pied en talus si bien que celui-ci ne peut jamais venir au contact du sol au cours de la marche. En conséquence, la chaussure selon l'invention permet à un patient ayant eu l'avant-pied opéré ou atteint d'affection, de marcher normalement sans ressentir de douleurs.

De préférence, la partie antérieure de la semelle pourra être conformée selon un coin amortisseur dirigé vers l'avant de la chaussure. Grâce à cette disposition, on augmente l'assise et la souplesse de la chaussure, ce qui confère au patient un bon équilibre au cours de la marche.

Selon une caractéristique importante de l'invention, la semelle est en outre compressible et présente un degré de compressibilité allant en augmentant de l'arrière vers l'avant de la semelle.

A cette fin, la semelle comporte un socle dur sur lequel reposent la première et la tige, la partie restante de la semelle se composant d'un matériau compressible homogène, tel qu'une mousse synthétique alvéolée, du latex ou du caoutchouc.

De préférence, le socle et la partie compressible de la semelle sont moulés en un seul bloc. En outre, la semelle porte sur sa surface de marche une plaque de protection et un matériau dur.

Grâce à la compressiblilité variable de la semelle, on évite un arrêt brutal de l'appui de l'arrière-pied au niveau de la partie antérieure de la chaussure, ce qui améliore considérablement le confort du patient au cours de la marche. En outre, le socle dur sert de protection anti-choc pour le pied.

Selon une autre caractéristique importante de l'invention, une embase est interposée entre la semelle et la première, cette embase se prolongeant au-delà du bord antérieur de la semelle par une languette flexible incurvée vers le bas, qui s'arrête selon une courbe correspondant à l'interligne de Lisfranc du pied du patient. Cette languette permet un déroulement partiel du pied au cours de la marche tout en laissant libre le métatarse.

Avantageusement, les deux éléments latéraux de la tige sont deux quartiers réalisés en une toile forte et sont d'une hauteur suffisante pour envelopper l'arrière-pied du patient jusqu'au dessus des malléoles péronéo-tibiales. La tige maintient ainsi parfaitement en place le pied du patient pour participer efficacement au positionnement en talus de ce dernier.

De préférence, les deux quartiers de la tige peuvent être reliés l'un à l'autre par des attaches auto-agrippantes, qui permettent un réglage de la fermeture de la tige en fonction de la grosseur du pied et notamment un réglage dans le temps en fonction de l'évolution de l'œdème post-opératoire, dans le cas d'une opération de l'avant-pied.

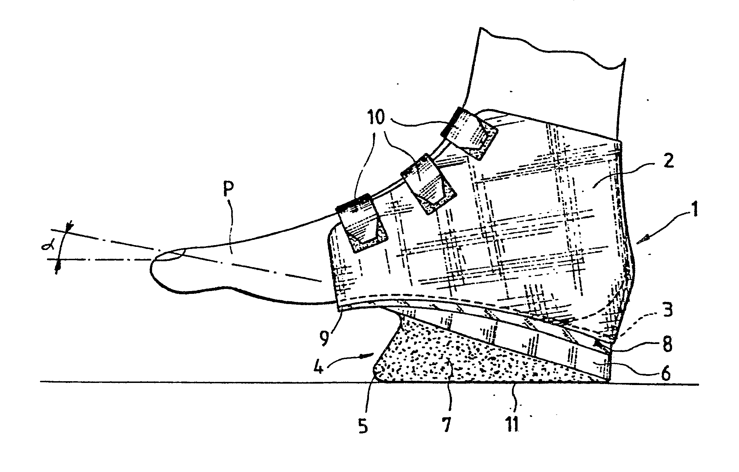

Un mode de réalisation de la présente invention va être décrit ci-après à titre d'exemple non-limitatif en référence à la figure unique du dessin annexé qui représente, en vue latérale, une chaussure post-opératoire selon l'invention en place autour du pied d'un patient.

Comme on peut le voir, cette chaussure comprend une tige 1 ouverte à l'avant, qui se compose de deux quartiers latéraux 2, dont un seul est visible sur la figure, lesdits quartiers se rejoignant à leurs parties inférieures de manière à former une première 3, ladite tige et ladite première reposant sur une semelle épaisse 4, nettement plus épaisse à l'avant qu'à l'arrière. Les quartiers 2 peuvent être refermés sur le cou-de-pied du pied P afin d'envelopper parfaitement la partie arrière de ce dernier.

De par cette structure particulière, la chaussure selon l'invention n'exerce aucun effort sur l'avant-pied et maintient le pied P en talus comme le représente l'angle α. Ainsi, l'avant-pied venant d'être opéré ne risque pas, au cours de la marche, de venir en contact avec le sol.

On observera que la partie antérieure de la semelle 4 est conformée selon un coin amortisseur 5 dirigé vers l'avant de la chaussure. Comme on le comprendra, cette disposition sert à augmenter l'assise et la souplesse de la chaussure pour conférer au patient un bon équilibre au cours de la marche.

Selon une caractéristique importante de l'invention, la semelle 2 est compressible, son degré de compressibilité allant en augmentant de l'arrière vers l'avant. Pour obtenir cette dernière propriété, on prévoit, dans la partie supérieure de la semelle, un socle dur 6 d'épaisseur décroissante de l'arrière vers l'avant, sur lequel repose la première 3 de la tige. La partie restante 7 de la semelle, de section longitudinale triangulaire, est formée d'un matériau compressible homogène, et plus précisément d'une mousse alvéolée. Le socle 6 et la partie compressible 7 peuvent être constitués par deux pièces distinctes accolées l'une à l'autre au cours de la fabrication de la chaussure. De préférence toutefois, on moule la semelle en un seul bloc à partir d'une résine synthétique, de latex ou de caoutchouc et selon un procédé connu qui permet un alvéolage partiel de la résine, du latex ou du caoutchouc.

On ajoutera ici que, pour des raisons de protection du matériau compressible, la semelle 4 comporte, sur sa surface de marche une plaque 11 en un matériau dur, cette plaque pouvant être obtenue par moulage monobloc avec les autres éléments constitutifs de la semelle.

Grâce à la compressibilité variable de la semelle 4, on évite un arrêt brutal de l'appui de l'arrière-pied au niveau de la partie antérieure de la chaussure, ce qui améliore considérablement le confort du patient au cours de la marche. Par ailleurs, le socle dur 6 constitue une protection anti-choc pour le pied.

La chaussure selon l'invention comporte en outre, interposée entre la première de tige 3 et la semelle 4, une embase 8 qui se prolonge au-delà du bord antérieur de la première de tige 3 par une languette flexible 9, incurvée vers le bas. Cette languette flexible permet un déroulement partiel du pied au cours de la marche. Par ailleurs, cette languette s'arrête le long d'une courbe correspondant à l'interligne de Lisfranc du pied P, laissant ainsi libre le métatarse qui n'est soumis de ce fait à aucun effort.

La tige 1 de la chaussure selon l'invention doit être suffisamment rigide pour participer au maintien du pied en talus. C'est pourquoi ses deux quartiers 2 sont réalisés en une toile forte renforcée par un contrefort et sont taillés sur une hauteur suffisante pour envelopper l'arrière-pied du patient jusqu'au dessus des malléoles péronéo-tibiales.

Comme on peut le voir, les quartiers 2 sont pourvus d'attaches auto-agrippantes 10 qui permettent de fermer la tige 1 autour du pied P. Ces attaches 10 sont au nombre de trois, l'une étant disposée au niveau des malléoles, la deuxième sur la partie antérieure de la tige et la troisième en position intermédiaire; elles constituent un moyen de fermeture de la tige réglable en fonction de la grosseur du pied. Plus précisément, après une opération chirurgicale au niveau de l'avant-pied, elles permettent un réglage en fonction de l'évolution de l'œdème post-opératoire.

La chaussure qui vient d'être décrite est essentiellement utilisée comme chaussure post-opératoire pour des patients ayant subi une opération chirurgicale au niveau de l'avant-pied, mais peut bien évidemment servir à chausser des patients atteints d'une affection traumatique de l'avant-pied.

Dans ces utilisations, elle offre la possibilité aux patients qui la porte, de marcher normalement au cours de leur convalescence, sans l'aide de béquilles ou de cannes, et ce, même s'ils sont obligés de porter une chaussure à chaque pied.

## Revendications

1. Chaussure, notamment chaussure post-opératoire pour patients ayant subi une intervention chirurgicale au niveau de l'avant-pied, cette chaussure étant ouverte à l'avant et comprenant une tige (1) comportant deux éléments latéraux (2) et une semelle (4) d'épaisseur inégale, caractérisée en ce que la tige (1) avec ses deux éléments latéraux (2) qui se rejoignent à leurs parties inférieures de manière à former une première (3) sont dimensionnés pour n'envelopper que l'arrière-pied du patient et reposent sur ladite semelle (4) qui est nettement plus épaisse à l'avant qu'à l'arrière de la tige (1).

2. Chaussure selon la revendication 1, caractérisée en ce que la partie antérieure de la semelle (4) est conformée selon un coin (5) dirigé vers l'avant de la chaussure.

3. Chaussure selon la revendication 1 ou 2, caractérisée en ce que la semelle (4) est compressible et présente un degré de compressibilité allant en augmentant de l'arrière vers l'avant.

4. Chaussure selon la revendication 3, caractérisée en ce que la semelle (4) comporte un socle dur (6) sur lequel reposent la tige (1) et la première, la partie restante (7) de la semelle (4) se composant d'un matériau compressible homogène.

5. Chaussure selon la revendication 4, caractérisée en ce que ledit matériau compressible homogène est constitué par une mousse alvéolée de résine synthétique, de latex ou de caoutchouc.

6. Chaussure selon la revendication 4 ou 5, caractérisée en ce que le socle (6) et la partie compressible (7) de la semelle (4) sont moulés en un seul bloc.

7. Chaussure selon l'une quelconque des revendications 1 à 6, caractérisée en ce qu'une embase (8) est interposée entre la semelle et la première, cette embase (8) se prolongeant au-delà du bord antérieur de la semelle par une languette flexible (9) incurvée vers le bas, qui s'arrête selon une courbe correspondant à l'interligne de Lisfranc.

8. Chaussure selon l'une quelconque des revendications 1 à 7, caractérisée en ce que les deux éléments latéraux (2) de la tige (1) sont deux quartiers qui sont réalisés en une toile forte et sont d'une hauteur suffisante pour envelopper l'arrière-pied jusqu'au dessus des malléoles péronéo-tibiales.

9. Chaussure selon la revendication 8, caractérisée en ce que les deux quartiers (2) de la tige (1) peuvent être reliés l'un à l'autre par des attaches auto-agrippantes (10).

10. Chaussure selon l'une quelconque des revendications 1 à 9, caractérisée en ce que la semelle (4) porte sur sa surface de marche une plaque de protection (11) en un matériau dur.

**Patentansprüche**

1. Schuh, insbesondere für Patienten, die sich einer Operation am vorderen Teil des Fusses unterzogen haben, vorne geöffnet und bestehend aus einem Schaft (1) mit zwei Seitenelementen (2) und einer Sohle (4) ungleicher Dicke, dadurch gekennzeichnet, dass der Schaft (1) mit seinen zwei Seitenelementen (2), welche sich an ihren Unterteilen verbinden, so dass sie eine Innensohle (3) bilden, derart zugeschnitten sind, dass sie nur den hinteren Teil des Patientenfusses umschliessen, und dass sie sich auf die Sohle (4) stützen, deren Dicke wesentlich grösser ist vorne als hinten am Schaft (1).

2. Schuh nach Anspruch 1, dadurch gekennzeichnet, dass der Vorderteil der Sohle (4) wie ein nach dem vorderen Teil des Schuhes gerichteter Keil (5) ausgebildet ist.

3. Schuh nach Anspruch 1 oder 2, dadurch gekennzeichnet, dass die Sohle (4) zusammendrückbar ist, und dass der Zusammendrückbarkeitsgrad von hinten nach vorne ansteigt.

4. Schuh nach Anspruch 3, dadurch gekennzeichnet, dass die Sohle (4) aus einem harten Sockel (6) besteht, auf welchem sich der Schaft (1) und die Innensohle stützen, wobei sich der restliche Teil (7) der Sohle (4) aus einem homogenen und zusammendrückbaren Material zusammensetzt.

5. Schuh nach Anspruch 4, dadurch gekennzeichnet, dass sich das homogene und zusammendrückbare Material aus Schaumkunstharz, Schaumgummi oder Schaumlatex zusammensetzt.

6. Schuh nach Anspruch 4 oder 5, dadurch gekennzeichnet, dass der Sockel (6) und der zusammendrückbare Teil (7) der Sohle (4) einen einzigen geformten Teil bilden.

7. Schuh nach einem der Ansprüche 1 bis 6, dadurch gekennzeichnet, dass ein Untersatz (8) zwischen Sohle und Innensohle liegt, wobei dieser Untersatz (8) über die vordere Kante der Sohle hinaus durch eine biegsame nach unten gerichtete Lasche (9) erweitert wird, welche nach einer der Lisfranc Zwischenlinie entsprechenden Kurve endet.

8. Schuh nach einem der Ansprüche 1 bis 7, dadurch gekennzeichnet, dass die zwei Seitenelemente (2) des Schaftes (1) zwei Seitenwände sind, die aus einem festen Leinen bestehen, und die genügend hoch sind, um den Hinterteil des Fusses bis über die Wadenbein-Schienbein-Fussknöcheln zu umschliessen.

9. Schuh nach Anspruch 8, dadurch gekennzeichnet, dass die zwei Seitenwände (2) des Schaftes (1) durch selbstgreifende Befestigungen (10) miteinander verbunden werden können.

10. Schuh nach einem der Ansprüche 1 bis 9, dadurch gekennzeichnet, dass die Sohle (4) auf ihrer Gangfläche mit einer Schutzplatte (11) aus hartem Material versehen ist.

**Claims**

1. A shoe, more particularly for patients having undergone a surgical operation on the fore-foot, which is opened at the front and which comprises an upper (1) with two side members (2) and a sole (4) with uneven thickness, characterized in that the upper (1) with its two side members (2) joining in their lower parts for forming an inner sole (3) are sized for wrapping round only the rear part of the patient's foot and rest on said sole (4) which is substantially thicker at the front than at the rear of the upper (1).

2. A shoe according to claim 1, characterized in that the front part of the sole (4) is shaped like a wedge (5) directed towards the front part of the shoe.

3. A shoe according to claim 1 or 2, characterized in that the sole (4) is compressible and has a degree of compressibility increasing from the rear to the front.

4. A shoe according to claim 3, characterized in that the sole (4) comprises a hard pad (6) on which rest the upper (1) and the inner sole (3), the remaining part (7) of the sole (4) being made of a homogeneous compressible material.

5. A shoe according to claim 4, characterized in that said homogeneous compressible material is made of a cellular synthetic foam, latex or rubber.

6. A shoe according to claim 4 or 5, characterized in that the pad (6) and the compressible part (7) of the sole (4) are integrally moulded.

7. A shoe according to any one of claims 1 to 6, characterized in that a base (8) is placed between the sole and the inner sole, this base (8) extending beyond the front edge of the sole by a downwardly curved flexible tongue (9), which stops along a curve corresponding to the Lisfranc interline.

8. A shoe according to any one of claims 1 to 7, characterized in that the two side members (2) of the upper (1) form two quarters made of strong

0 077 713

cloth and are of sufficient height to wrap round the rear part of the foot up to above the peroneotibial malleoli.

9. A shoe according to any one of claims 1 to 8, characterized in that the two quarters (2) of the upper (1) can be connected to each other by self-gripping fasteners (10).

10. A shoe according to any one of claims 1 to 9, characterized in that the sole (4) carries on its walking surface a protection plate (11) made of a hard material.